# EUROPÄISCHE PATENTSCHRIFT

(11) **EP 2 650 252 B1**
(45) Veröffentlichungstag und Bekanntmachung des Hinweises auf die Patenterteilung: **25.02.2015**
(21) Anmeldenummer: 12194893.9
(22) Anmeldetag: 29.11.2012
(51) Int. Cl.: B67B 3/00

(54) **Behältnissterilisation mit UV-Strahlung**
Container sterilization with UV radiation
Stérilisation de récipients par rayonnement UV

(30) Priorität: 12.12.2011 DE 102011056260
(43) Veröffentlichungstag der Anmeldung: 16.10.2013
(73) Patentinhaber: Krones AG, 93073 Neutraubling (DE)
(72) Erfinder: Frankenberger, Günter, 93073 Neutraubling (DE); Engelhard, Patrick, 93073 Neutraubling (DE); Koller, Stefan, 93073 Neutraubling (DE)
(74) Vertreter: Bittner, Bernhard

(56) Entgegenhaltungen:
- EP-A2- 1 120 121
- WO-A2-2008/015358
- DE-A1- 19 520 925
- DE-A1-102009 043 726

## Beschreibung

Die vorliegende Erfindung bezieht sich auf eine Vorrichtung und ein Verfahren zum Sterilisieren von Behältnissen. Im Bereich der getränkeherstellenden Industrie ist es seit langem bekannt, die Behältnisse, welche beispielsweise mit einem Getränk befüllt werden sollen, vor der Abfüllung zu sterilisieren. Gerade für die besonders bedeutsame Innensterilisation dieser Behältnisse sind dabei unterschiedliche Verfahren bekannt. So ist es bekannt, die Behältnisse mittels chemischer Sterilisationsmittel wie beispielsweise Wasserstoffperoxid zu beaufschlagen. Daneben ist man aber in jüngerer Zeit auch dazu übergegangen, eine Sterilisation der Behältnisse in anderer Weise durchzuführen wie beispielsweise durch Elektronenstrahlung. Daneben ist es aus dem Stand der Technik aber auch bekannt, Behältnisse mittels UV-Strahlung zu sterilisieren.

EP 1120121 A2 offenbart eine stabförmige UV-Desinfektionsvorrichtung für Flaschen gleichkommt, wobei diese stabförmige UV-Desinfektionsvorrichtung in die Flasche axial einführbar ausgeführt ist.

So beschreibt beispielsweise die DE 10 2004 032861 A1 ein Verfahren und eine Vorrichtung zum Sterilisieren von Behältern mit UV-Strahlung. Dabei wird die UV-Strahlung einer vollständig außerhalb des Behälters befindlichen UV-Quelle über ein durch eine Behältermündung in den Behälterinnenraum eingeführtes Lichtleiterelement ausgestrahlt.

Die DE 10 2009 043496 A1 beschreibt ein Verfahren und eine Vorrichtung zur Sterilisation von Oberflächen. In dieser Druckschrift wird vorgeschlagen, dass die Strahlung von einer Strahlungsquelle bis zur Oberfläche durch ein auf die Strahlung abgestimmtes Transmissionsmedium geführt wird. Bei diesem Transmissionsmedium kann es sich beispielsweise um ein Gas, insbesondere ein Schutzgas und dergleichen, handeln.

Die DE 10 2009 043726 A1 beschreibt ebenfalls ein Verfahren und eine Vorrichtung zur Sterilisation von Oberflächen. Hierbei wird kurzwellige elektromagnetische Strahlung eingesetzt, welche die Oberfläche beaufschlagt, wobei auch hier die Strahlung von einer Strahlungsquelle bis zu der Oberfläche durch ein Transmissionsmedium geführt wird.

Auch aus der EP 1 120 121 A2 ist ein Sterilisationssystem basierend auf UV-Strahlung bekannt. Dabei ist eine Vielzahl von elektrodenlosen Plasmalampen vorgesehen, welche die UV-Strahlung ausgeben.

Untersuchungen mit UV-Lampen zeigten bei einem Entkeimungsprozess Schwierigkeiten, eine ausreichende Gasatmosphäre, beispielsweise eine ausreichende N₂ - Atmosphäre in dem Behältnis zur prozesssicheren Entkeimung aufrechtzuerhalten. Ein Spülen des Behältnisses mit N₂ (Stickstoff) unmittelbar vor dem Eintauchen der Strahlungsquelle reicht dabei nicht aus, da sofort nach der Begasung ein Gasaustausch mit der Umgebungsluft stattfindet. Die für den Prozess erforderliche Strahlungsleistung an der Behälterinnenseite konnte im Versuch nur durch eine permanente N₂ -Zufuhr erzielt werden. Aufgrund des kleinen Behältnisdurchmessers und andererseits des vergleichsweise großen Lampendurchmessers ergab sich jeweils nur ein minimaler Ringspalt, der für eine effektive Spülung problematisch ist.

Der vorliegenden Erfindung liegt daher die Aufgabe zugrunde, eine Sterilisation mit einer verbesserten Spülung des Behältnisses durchzuführen. Diese Aufgaben werden erfindungsgemäß durch die Gegenstände der unabhängigen Ansprüche erreicht. Vorteilhafte Ausführungsformen und Weiterbildungen sind Gegenstand der Unteransprüche.

Eine erfindungsgemäße Vorrichtung zum Sterilisieren von Behältniskomponenten ist in Anspruch 1 definiert.

Erfindungsgemäß weist das Strahlungselement eine Leitungseinrichtung zum Leiten eines fließfähigen und insbesondere gasförmigen Mediums auf, sowie wenigstens eine Öffnung, mittels der das fließfähige Medium zwischen dem Strahlungselement und dem Inneren des Behältnisses (bzw. Behältnisverschlusses bzw. allgemein der Behältniskomponente) strömen kann bzw. geleitet werden kann. Die Leitungseinrichtung ist dabei vorzugsweise als Kanal ausgebildet, durch den das fließfähige Medium strömen kann bzw. geleitet werden kann.

Damit wird vorgeschlagen, dass über die besagte Öffnung ein fließfähiges Medium entweder von dem Stangenelement in das Innere des Behältnisses (bzw. Behältnisverschlusses) einbringbar ist, oder umgekehrt, von dem Behältnisinneren (bzw. dem Inneren des Behältnisverschlusses) in das Stangenelement führbar ist. Bevorzugt weist die Vorrichtung auch eine Bewegungseinrichtung auf, um das Stangenelement bezüglich des Behältnisses (bzw. Behältnisverschlusses) in einer Längsrichtung des Behältnisses bzw. Behältnisverschlusses zu bewegen. Im folgenden wird zur Vereinfachung lediglich auf Behältnisse Bezug genommen. Es wird jedoch darauf hingewiesen, dass eine entsprechende Anwendung auch jeweils, wie oben dargestellt, auf Behältnisverschlüsse möglich ist.

Dabei ist es möglich, dass die Stangenelemente in der Längsrichtung der Behältnisse stationär angeordnet sind und eine Hubeinrichtung vorgesehen ist, welche die Behältnisse in ihrer Längsrichtung anhebt, sodass das Stangenelement in das Innere der Behältnisse eindringt. Vorteilhaft transportiert die Transporteinrichtung auch die Behältnisse selbst entlang des vorgegebenen Transportpfades. Zu diesem Zweck kann die Transporteinrichtung auch Halteeinrichtungen aufweisen, welche die Behältnisse während ihres Transports halten. Die Transporteinrichtung kann einen drehbaren Träger aufweisen, an dem diese Halteeinrichtungen angeordnet sind.

Vorteilhaft handelt es sich bei dem fließfähigen Medium um ein gasförmiges Medium, wie insbesondere aber nicht ausschließlich Stickstoff (N₂). Die vorliegende Erfindung ist prinzipiell auch zur Sterilisierung von Behältniskomponenten geeignet, wie insbesondere aber nicht ausschließlich von Behältnisverschlüssen. Damit wird hier eine Strahlungsquelle in Form einer stabförmigen UV-Lampe zur Sterilisation des Behältnisses oder Verschlusses vorgeschlagen, welche mit einer Bohrung zur Führung des Mediums versehen ist. Diese Bohrung kann zur Einleitung eines Inertgases verwendet werden, welches während der Entkeimung einen aktiven Spülprozess bzw. die N₂ -Konzentration des fließfähigen Mediums innerhalb des Behältnisses aufrecht erhält.

Auch kann die Öffnung bzw. Bohrung zur Einleitung von Flüssigkeiten oder Gasen oder Gasgemischen verwendet werden, welche durch die Strahlung aktiviert werden und ihrerseits den Sterilisationsprozess durchführen. Diese könnten sowohl in der Behältnisatmosphäre verbleiben oder sich auch an der Innenwandung anlegen bzw. auskondensieren.

Bei einer weiteren vorteilhaften Ausführungsform weist die Vorrichtung eine Vielzahl von Halteeinrichtungen auf, welche die einzelnen Behältnisse halten. Dabei können beispielsweise Greifelemente vorgesehen sein, welche die Behältnisse unterhalb ihres Tragrings halten und so beispielsweise auch bevorzugt heben und absenken können. Vorteilhaft ist dabei jede Halteeinrichtung einem Strahlungselement zugeordnet.

Bei einer weiteren vorteilhaften Ausführungsform weist die Vorrichtung ein Reservoir für ein weiteres fließfähiges Medium, z. B. Dampf, auf. So kann beispielsweise durch die Öffnung zuerst Dampf eingeführt werden, der an der Behältnisinnenwandung kondensiert und sich positiv auf den anschließenden Entkeimungsprozess auswirkt. Auch wäre es möglich, dass das Behältnis kopfüber entkeimt wird. In diesem Falle kann vor der Entkeimung durch die Öffnung ein aktiver Spülvorgang geschalten werden. Diese Spülung kann dabei mit Flüssigkeit oder mit Druckluft erfolgen. Vorteilhaft ist es möglich, dass auch mehrere verschiedene hintereinandergeschaltete Einbring- und Spülvorgänge durchgeführt werden.

Bei einer weiteren vorteilhaften Ausführungsform ist die Leitungseinrichtung als wenigstens abschnittsweise im Inneren des Strahlungselements verlaufender Kanal ausgebildet. Dieser Kanal kann dabei beispielsweise zentrisch im Inneren des Strahlungselements geführt werden. Es wäre jedoch auch eine außermittige oder wendelförmige Ausgestaltung dieses Kanals möglich. Wie erwähnt, dient dieser Kanal insbesondere zur Führung von Inertgasen. Daneben können jedoch auch Gase oder Flüssigkeiten geführt werden, die durch die Strahlungsquelle aktiviert werden können, um die Entkeimungswirkung zu verbessern oder zu ermöglichen.

Bei einer weiteren vorteilhaften Ausführungsform ist ein Querschnitt des Strahlungselements derart dimensioniert, dass bei einem in das Behältnis eingeführten Strahlungselement zwischen einer Außenwandlung des Strahlungselements und einer Wandlung des Behältnisses ein Spalt ausgebildet wird, durch welchen hindurch das fließfähige Medium zwischen einem Innenraum des Behältnisses und einem bezüglich des Behältnisses außenliegenden Raum strömen kann. Auf diese Weise ist es beispielsweise möglich, dass das fließfähige Medium durch das Strahlungselement in das Behältnis eingeführt wird und durch den besagten seitlichen Spalt wieder aus dem Behältnis ausgeführt wird. Dabei kann dieser Spalt beispielsweise als ringförmiger Spalt ausgebildet sein, d. h. das Strahlungselement weist hier bevorzugt einen im wesentlichen kreisförmigen Querschnitt auf, der jedoch geringer ist, als der Innenquerschnitt der Mündung des Behältnisses. Vorteilhaft ist der Durchmesser des Strahlungselements wenigstens 2 Millimeter kleiner als der Innendurchmesser der Behältnismündung, bevorzugt wenigstens 4 Millimeter kleiner und besonders bevorzugt wenigstens 8 Millimeter kleiner.

Bei einer weiteren vorteilhaften Ausführungsform ist wenigstens eine (Austritts- bzw. Eintritts-) Öffnung in einer Umfangsfläche des Strahlungselements angeordnet. So wäre es möglich, dass beispielsweise ein zentraler Kanal vorgesehen ist, der mehrere seitliche Austritte bzw. Öffnungen mit dem fließfähigen Medium versorgt und durch diese seitlichen Austritte das fließfähige Medium aus dem Strahlungselement austreten kann. Dabei können diese mehreren Öffnungen in einer Umfangsrichtung des Strahlungselements verteilt angeordnet sein. Daneben und/oder zusätzlich können die Öffnungen auch in einer Längsrichtung des Strahlungselements verteilt sein. Auf diese Weise ist es möglich, dass bei vollständig in das Behältnis eingeführtem Strahlungselement eine flächendeckende Bestrahlung der Innenwandung des Behältnisses erfolgt.

Daneben wäre es auch möglich, dass sich Querschnitte der einzelnen Öffnungen in der Längsrichtung des Strahlungselements 22 von oben nach unten ändern, beispielsweise diese Öffnungen von oben nach unten größer werden. Auf diese Weise kann erreicht werden, dass ein gasförmiges Medium im Wesentlichen mit gleichen Strömungsquerschnitten aus den einzelnen Öffnungen austritt.

Es wäre jedoch auch möglich, dass eine Öffnung stirnseitig an dem Strahlungselement 22 angeordnet ist und so das fließfähige Medium nahe an dem Boden des Behältnisses austritt bzw. in das Strahlungselement 22 eintritt. Auch wäre es möglich, dass sowohl eine stirnseitige Öffnung als auch eine oder mehrere Umfangsöffnungen vorgesehen sind.

Bei einer weiteren vorteilhaften Ausführungsform besitzt das Strahlungselement zwei oder auch mehrere vorteilhaft parallele Kanäle, welche in allen beschriebenen Varianten kombinierbar sein können. Dadurch können unterschiedliche Medien unabhängig zu unterschiedlichen Zeiten zugeführt oder abgesaugt werden.

Bei einer weiteren vorteilhaften Ausführungsform ist zumindest der Transportpfad der Behältnisse innerhalb eines Reinraums angeordnet. Dabei ist es möglich, dass dieser Reinraum ringförmig um den Transportpfad der Behältnisse ausgebildet ist. Auf diese Weise kann die Sterilisation bereits unter Reinraumbedingungen stattfinden. Weiterhin kann eine Dichtungseinrichtung vorgesehen sein, welche diesem Reinraum gegenüber einer Umgebung abdichtet. Zu diesem Zweck können dabei Dichtungseinrichtungen vorgesehen sein, welche wenigstens einen stehenden und einen gegenüber diesem stehenden beweglichen, insbesondere drehenden, Anteil aufweisen. Damit ist vorteilhaft die Transporteinrichtung als um eine vorgegebene Achse drehbarer Träger ausgeführt. Bei der Dichtungseinrichtung kann es sich um eine hydraulische Dichtung wie etwa ein sog. Wasserschloss handeln.

Bei einer weiteren vorteilhaften Ausführungsform ist an wenigstens einem Strahlungselement ein dieses Strahlungselement wenigstens abschnittsweise umgebender Hülsenkörper angeordnet. Dabei weist vorteilhaft dieser Hülsenkörper einen größeren Querschnitt auf, als die Mündung des Behältnisses. Vorteilhaft ist damit dieser hülsenförmige Körper auf die Mündung des Behältnisses aufsetzbar. Dabei ist es möglich, dass zwischen diesem hülsenförmigen Körper und dem Strahlungselement ebenfalls ein Spalt ausgebildet wird, durch welchen das fließfähige Medium oder auch ein anderes fließfähiges Medium geleitet werden kann. So ist es denkbar, dass durch den hülsenförmigen Körper der über der freien Oberfläche des Strahlungselements angeordnet ist, ein Inertgas nach dem Verlassen des Behältnisses hindurchgeführt wird, um bei der Entkeimung von kürzeren Behältnissen mit dem gleichen

Strahlungselement arbeiten zu können und dabei auch die Entstehung von Ozon zu verhindern oder zu verringern, welches Ozon entstünde, wenn die UV-Strahlung des freien Lampenbereichs in die Umgebungsatmosphäre strahlt.

Vorteilhaft kann dabei zwischen dem Hülsenkörper und dem Strahlungselement ebenfalls ein ringförmiger Spalt ausgebildet sein, durch den hindurch das fließfähige Medium führbar ist.

Die vorliegende Erfindung ist weiterhin auf eine Einrichtung zum Sterilisieren von Behältnissen oder Behältniskomponenten gerichtet, welche ein Strahlungselement mit einer Strahlungsquelle zur Abgabe von ultraviolettem Licht aufweist. Erfindungsgemäß weist das Strahlungselement eine kanalartig innerhalb des Strahlungselements angeordnete Leitungseinrichtung zum Leiten eines fließfähigen Mediums auf, sowie eine Zuführöffnung, über welche das fließfähige Medium der Leitungseinrichtung zugeführt werden kann, und eine Austrittsöffnung, durch die das fließfähige Medium aus dem Strahlungselement austreten kann. In dieser Gestaltung kann das erfindungsgemäße Strahlungselement auch zum Sterilisieren von anderen Behältniskomponenten wie beispielsweise Verschlüssen verwendet werden. Vorteilhaft weist jedoch das Strahlungselement eine stangenartige Gestalt auf und ist insbesondere in ein zu sterilisierendes Behältnis einführbar.

Die vorliegende Erfindung ist weiterhin auf ein Verfahren zum Sterilisieren von Behältnissen nach Anspruch 7 gerichtet.

Erfindungsgemäß wird dem Innenraum der Behältnisse ein fließfähiges Medium zugeführt, und das Strahlungselement weist eine Leitungseinrichtung auf, mittels der dieses fließfähige Medium transportiert wird. Dabei wird bevorzugt durch diese Leitungseinrichtung und eine Austrittsöffnung das fließfähige Medium in die Behältnisse eingeführt.

Vorteilhaft wird das fließfähige Medium auch zwischen einer Außenwandlung des Strahlungslements und eine Innenwandlung der Mündung des Behältnisses tranpsortiert und auf diesem Weg bevorzugt aus dem Behältnis abgeführt.

Auch das erfindungsgemäße Verfahren ist nicht nur auf die eigentlichen Behältnisse sondern allgemein auf Behältniskomponenten anwendbar, wie insbesondere auch Behältnisverschlüsse.

Weiterhin wäre es auch möglich, frisches Gas zur unterstützenden Kühlung des Behältnisses, einer Behältniskomponente, oder auch der Lampe selbst zuzuführen.

Weitere Vorteile und Ausführungsformen ergeben sich aus den beigefügten Zeichnungen:
Darin zeigen:
   - Figur 1:: eine schematische Darstellung einer erfindungsgemäßen Vorrichtung zum Sterilisieren von Behältnissen;
   - Figur 2:: eine schematische Darstellung einer erfindungsgemäßen Vorrichtung in einer ersten Ausführungsform;
   - Figur 3:: eine schematische Darstellung einer erfindungsgemäßen Vorrichtung in einer zweiten Ausführungsform; und
   - Figur 4:: eine schematische Darstellung einer erfindungsgemäßen Vorrichtung in einer dritten Ausführungsform.
   - Figur 5a-b:: zwei schematische Darstellungen einer erfindungsgemäßen Vorrichtung in einer Ausführungsform für Verschlüsse.

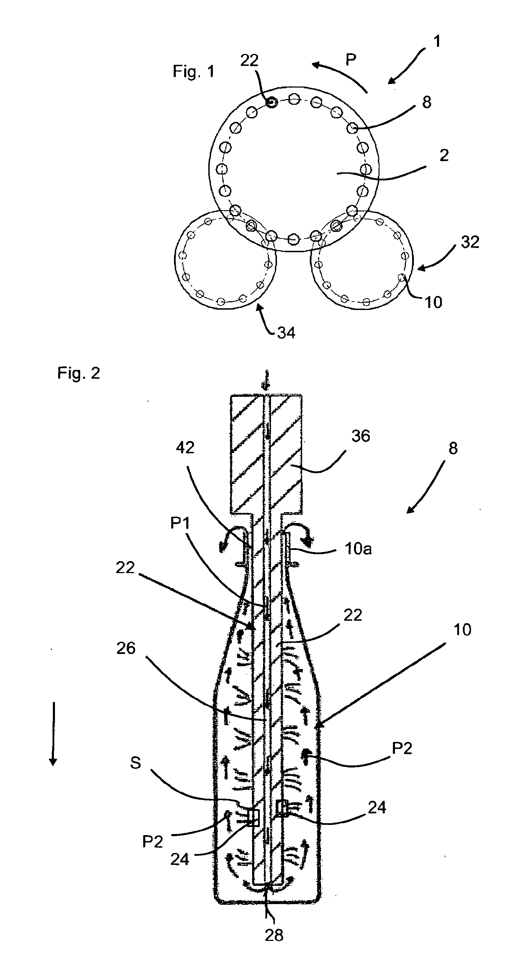

Figur 1 zeigt eine schematische Darstellung einer erfindungsgemäßen Vorrichtung 1 zum Sterilisieren von Behältnissen 10. Dabei werden die Behältnisse 10 über eine Zuführeinrichtung 32 wie beispielswiese ein Zuführrad einer Transporteinrichtung 2 zugeführt. Diese Transporteinrichtung 2 ist hier als ein drehbarer Träger ausgebildet, an dem eine Vielzahl von Sterilisationseinheiten 8 angeordnet ist. Den einzelnen Sterilisationseinheiten 8 werden jeweils die Behältnisse 10 übergeben und während des Transports der Behältnisse entlang des Transportpfades P findet die Sterilisation statt. Zu diesem Zweck tauchen Strahlungselemente 22 (nur eines dargestellt) durch die Mündungen der Behältnisse in diese Behältnisse ein. Nach dem Sterilisationsvorgang werden die Behältnisse über ein Abführrad 34, bei dem es sich ebenfalls wieder um einen Abführstern handeln kann, abgeführt.

Figur 2 zeigt eine schematische Darstellung einer Sterilisationseinheit 8. Diese Sterilisationseinheit weist dabei ein stangenartiges Strahlungselement 22 auf, welches über eine Mündung 10a hin in die Behältnisse 10 einführbar ist. Dieses Strahlungselement 22 weist eine Vielzahl von Strahlungsquellen 24 an ihrem Außenumfang auf, welche jeweils eine ultraviolette Strahlung S abgeben. Dabei ist es möglich, dass ultraviolette Strahlungsquellen 24 direkt an dem Strahlungselement 22 angeordnet sind. Es wäre jedoch auch möglich, dass eine zentrale oder mehrere zentrale Strahlungsquellen vorgesehen sind, beispielsweise an oder in dem Träger 36 und die UV-Strahlung mittels Lichtleitern zu den einzelnen Strahlungsquellen 24 geleitet wird.

Weiterhin weist das Strahlungselement 22 eine radial innenliegende Leitungseinrichtung 26 auf, welche zum Leiten eines gasförmigen Mediums, beispielsweise von Stickstoff (N₂) dient. Diese Leitungseinrichtung ist hier in der Art eines sich in der Längsrichtung L des Strahlungselements erstreckenden Kanals ausgebildet und endet in einer Öffnung 28 am unteren Ende des Strahlungselements. Über diese Öffnung kann das gasförmige Medium aus dem Strahlungselement 22 austreten und in das Innere des Behältnisses gelangen.

Man erkennt, dass bei der in Figur 2 gezeigten Ausführungsform das fließfähige Medium innerhalb der Leitungseinrichtung von oben nach unten (Pfeil P1) geführt wird, anschließend durch die Öffnung 28 austritt und zwischen dem Strahlungselement und der Innenwandung des Behältnisse (Pfeil P2) wieder nach oben gelangt.

Weiterhin erkennt man, dass zwischen dem Außenumfang des Strahlungselements 22 und dem Innenumfang des Behältnisses ein Spalt 42 ausgebildet wird, durch den hindurch das fließfähige Medium strömen kann.

Wie bereits oben erwähnt, kann die Leitungseinrichtung 26 zur Einleitung eines Inertgases verwendet werden, welches während der Entkeimung einen aktiven Spülprozess und/oder die N₂ - Konzentration aufrechterhält.

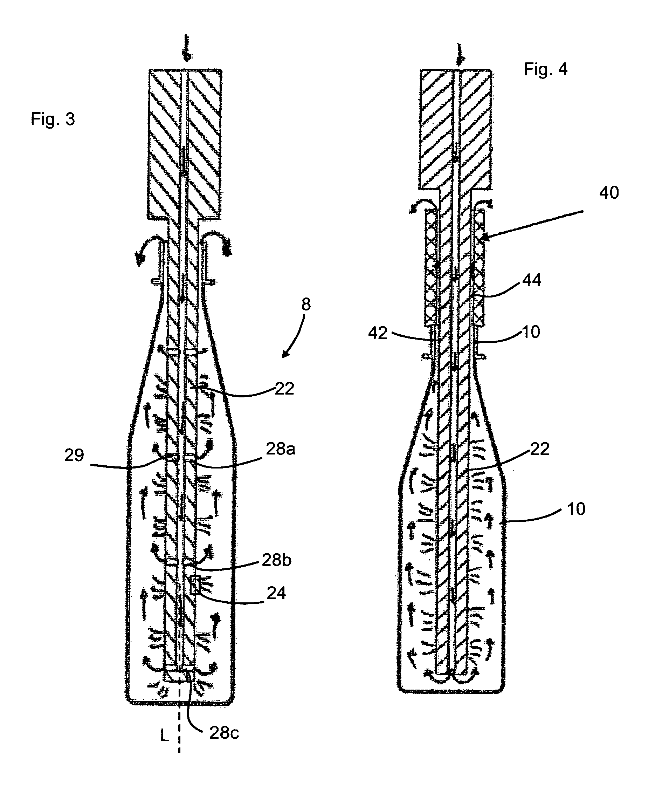

Figur 3 zeigt eine weitere Ausführungsform einer erfindungsgemäßen Sterilisationseinheit 8. Bei dieser Ausführungsform ist eine Vielzahl von am Außenumfang des Strahlungselements angeordneten Öffnungen 28a, 28b und 28c vorgesehen, welche ebenfalls von der Leitungseinrichtung 26 versorgt werden, jedoch hier mit dieser Leitungseinrichtung 26 über Querkanäle 29 in Strömungsverbindung stehen. Damit tritt bei dieser Ausführungsform das fließfähige Medium über die im Wesentlichen gesamte Länge des Strahlungselements 22 an dessen Außenumfang aus.

Zwischen den einzelnen Öffnungen 28a, 28b und 28c sind wieder die Strahlungsquellen 24 angeordnet, welche vollumfänglich Strahlung abgeben. Es wäre zusätzlich denkbar, dass eine Dreheinrichtung vorgesehen ist, welche das Behältnis 10 bezüglich der Längsrichtung L dreht, oder welche das Strahlungselement 22 um die besagte Längsrichtung L dreht. In diesem Falle wäre es denkbar, dass nur an einem Bereich in Umfangsrichtung des Strahlungselements 22, Strahlungsquellen 24 angeordnet sind.

Figur 4 zeigt eine Darstellung einer erfindungsgemäßen Vorrichtung in einer weiteren Ausführungsform. Diese Ausführungsform kann beispielsweise für kürzere Behältnisse 10 verwendet werden. Zusätzlich zu den oben beschriebenen Ausführungsformen ist hier ein hülsenartiger Körper 40 vorgesehen, der während der Sterilisationsvorgangs an die Mündung 10a des Behältnisses 10 angelegt werden kann. In diesem Falle kann das fließfähige Medium zunächst durch den Spalt 42 strömen und dann durch einen weiteren Spalt 44, der zwischen dem hülsenförmigen Körper 40 und dem Strahlungselement 22 ausgebildet wird.

Durch diesen Spalt 44 kann das Inertgas nach dem Verlassen des Behältnisses geführt werden, sodass bei der Entkeimung von kürzeren Behältnissen 10 mit demselben Strahlungselement die Entstehung von Ozon im Behältnis, aber auch über der Behältnismündung minimiert werden kann. Auf diese Weise ist es denkbar, dass das gleiche Strahlungselement 22 für unterschiedliche Behälterlängen verwendet wird. Dieser hülsenförmige Körper 40 kann dabei beispielsweise zuschaltbar sein, etwa indem er in eine Bewegung des Strahlungselements gefahren wird, bzw. über das Strahlungselement 22 gestülpt wird.

Es wäre auch möglich, dass der hülsenartige Körper 40 in Form eines klammerartigen Elements ausgeführt ist, welches um das Strahlungselement 22 geschlossen und geöffnet werden kann.

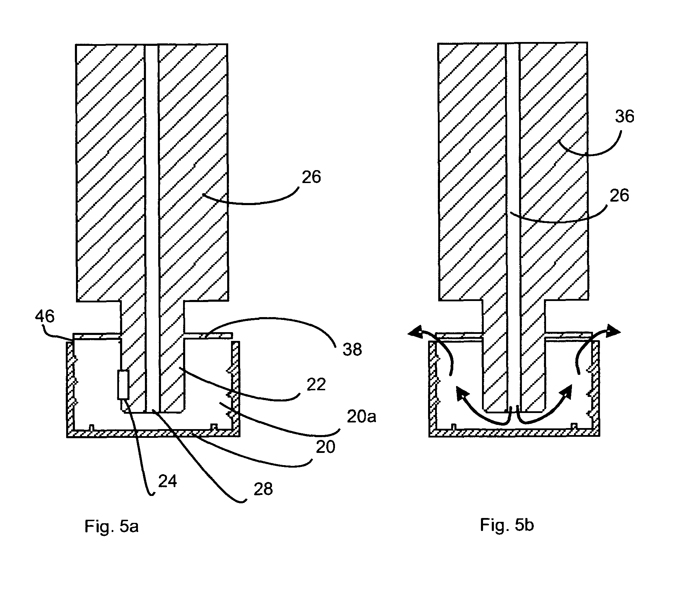

Die Figuren 5a, b zeigen die Anwendung der Erfindung zum Sterilisieren von Behältnisverschlüssen 20. Hier ist ebenfalls ein stangenartig ausgebildetes Strahlungselement 22 vorgesehen, welches hier jedoch wesentlich kürzer ist als das Strahlungselement, welches in die Behältnisse 10 eingeführt wird. Dieses Strahlungselement 22 wird ebenfalls in den Innnenraum 20a der Vehältnisverschlüsse eingeführt. Mittels der Strahlungseinrichtung 24 erfolgt wiederum eine Sterilisierung der Innenwandung der Behältnisverschlüsse 20. Daneben ist auch wieder eine Leitungseinrichtung 26 zum Leiten des gasförmigen Mediums vorgesehen sowie eine Öffnung 28, über welche das gasförmige Medium aus dem Strahlungselement 22 austritt. Bei der Anwendung für Behältnisverschlüsse befindet sich die Öffnung 28 vorzugsweise stirnseitig an dem Strahlungselement 22.

Weiterhin weist die in Fig. 5a gezeigte Vorrichtung noch ein Abdeckelement 38 auf, welches die Öffnung 20b des Behältnisverschlusses 20 weitgehend abdeckt. Genauer gesagt ergibt sich noch ein umlaufender Spalt 46, durch den das gasförmige Medium aus dem Behältnisverschluss austreten kann. In Fig. 5b sind durch die Pfeile die Strömungsverläufe dargestellt. Der Spalt kann hier dadurch entstehen, dass das Abdeckelement hinsichtlich seines Querschnitts geringfügig kleiner ist als der Behältnisverschluss. Es wäre jedoch auch möglich, dass das Abdeckelement genauso groß oder sogar größer (im Querschnitt) ist als der Behältnisverschluss, das Abdeckelement jedoch nicht ganz auf den Behältnisverschluss aufgesetzt wird und sich so ein Spalt oberhalb des Behältnisverschlusses ergibt.

Es wäre jedoch auch möglich, dass das Abdeckelement den Behältnisverschluss vollständig abdeckt und innerhalb des Abdeckelements 38 Schlitze oder Öffnungen ausgebildet sind, durch welche das gasförmige Medium austreten (oder gegebenenfalls eintreten) kann. Weiterhin wäre es auch denkbar, dass die Position des Abdeckelements 38 gegenüber dem Strahlungselement 22 in der Längsrichtung des Strahlungselements veränderbar ist, um so in einfacher Weise eine Umstellung auf unterschiedliche Höhen von Behältnisverschlüssen zu ermöglichen. Das Abdeckelement 38 erstreckt sich bevorzugt, wenigstens teilweise, senkrecht zu der Längsrichtung des Strahlungselements 22 und ist bevorzugt platten-, scheiben- oder glockenartig ausgebildet. Vorteilhaft weist das Abdeckelement 38 eine kreisförmige Gestalt, wobei bei einer glockenartigen Ausgestaltung der von einem scheibenförmigen Teil ausgehende Glockenrand in Richtung des zu behandelnden Gegenstandes weist und einen Innendurchmesser aufweist, der größer ist als der Außendurchmesser der Öffnung des zu behandelnden Gegenstandes (beispielsweise Behältnismündung).

### Bezugszeichenliste

- 1: Vorrichtung zum Sterilisieren von Behältnissen
- 2: Transporteinrichtung
- 8: Sterilisationseinheiten
- 10: Behältnisse
- 10a: Mündung der Behältnisse
- 20: Behältnisverschluss
- 20a: Innenraum des Behältnisverschlusses
- 20b: Öffnung des Behältnisverschlusses
- 22: Strahlungselement
- 24: Strahlungsquelle
- 26: Leitungseinrichtung
- 28: Öffnung (stirnseitig)
- 28a, 28b, 28c: Öffnungen (umfangsseitig)
- 32: Zuführrad
- 34: Abführrad
- 36: Träger
- 40: hülsenartiger Körper
- 42: Spalt
- 44: weiterer Spalt
- 46: Spalt

- S: Strahlung
- P: Transportpfad
- P1, P2: Pfeile
- L: Längsrichtung des Behältnisses

## Patentansprüche

1. Vorrichtung (1) zum Sterilisieren von Behältniskomponenten (10, 20) mit einer Transporteinrichtung (2), welche eine Vielzahl von Sterilisationseinheiten (8) entlang eines vorgegebenen Transportpfades (P) transportiert, wobei jede dieser Sterilisationseinheiten (8) ein stangenartig ausgebildetes Strahlungselement (22) aufweist, welches wenigstens teilweise durch eine Mündung (10a) der Behältniskomponente (10, 20) in die Behältniskomponente einführbar ist, und wobei das Strahlungselement (22) wenigstens eine Strahlungsquelle (24) zur Abgabe von ultraviolettem (UV) Licht aufweist,
**dadurch gekennzeichnet, dass**
das Strahlungselement eine Leitungseinrichtung (26) zum Leiten eines fließfähigen Mediums aufweist sowie wenigstens eine Öffnung (28, 28a, 28b..) mittels der das fließfähige Mittel zwischen dem Strahlungselement (22) und dem Inneren der Behältniskomponente (10, 20) strömen kann, wobei die Leitungseinrichtung (26) wenigstens abschnittsweise als ein im Inneren des Strahlungselements (22) verlaufender Kanal ausgebildet ist.

2. Vorrichtung (1) nach wenigstens einem der vorangegangenen Ansprüche,
**dadurch gekennzeichnet, dass** ein Querschnitt des Strahlungselements (22) derart dimensioniert ist, dass bei in die Behältniskomponente (10,20) eingeführtem Strahlungselement zwischen einer Außenwandung des Strahlungselements (22) und einer Wandung der Behältniskomponente (10,20) ein Spalt (42) ausgebildet wird, durch welchen hindurch das fließfähige Medium zwischen einem Innenraum der Behältniskomponente (10, 20) und einem bezüglich der Behältniskomponente (10, 20) außen liegenden Raum strömen kann.

3. Vorrichtung (1) nach Anspruch 3, **dadurch gekennzeichnet, dass** der Spalt (42) ein ringförmiger Spalt ist.

4. Vorrichtung (1) nach wenigstens einem der vorangegangenen Ansprüche, **dadurch gekennzeichnet, dass** wenigstens eine Öffnung (28a, 28b) in einer Umfangsfläche des Strahlungselements (22) angeordnet ist.

5. Vorrichtung (1) nach wenigstens einem der vorangegangenen Ansprüche, **dadurch gekennzeichnet, dass** an wenigstens einem Strahlungselement (22) ein dieses Strahlungselement (22) wenigstens abschnittsweise umgebender Hülsenkörper (40) angeordnet ist.

6. Vorrichtung (1) nach Anspruch 5, **dadurch gekennzeichnet, dass** zwischen dem Hülsenkörper (40) und dem Strahlungselement (22) ein Spalt (44) ausgebildet ist, durch den das fließfähige Medium führbar ist.

7. Verfahren zum Sterilisieren von Behältniskomponenten (10,20), wobei die Behältniskomponenten (10,20) mittels einer Transporteinrichtung (2) transportiert werden und während dieses Transports mittels einer Vielzahl von Sterilisationseinheiten (8) sterilisiert werden, wobei diese Sterilisationseinheiten (8) jeweils Strahlungselemente (22) aufweisen, die über eine Mündung der Behältniskomponenten (10, 20) in die Behältniskomponenten (10,20) eingeführt werden und wobei diese Strahlungselemente (22) im Innenraum der Behältniskomponenten (10,20) eine ultraviolette Strahlung zum Sterilisieren der Innenwandung der Behältniskomponenten (10,20) abgeben,
**dadurch gekennzeichnet, dass**
dem Innenraum der Behältniskomponenten (10,20) ein fließfähiges Medium zugeführt wird und das Strahlungselement (22) eine Leitungseinrichtung (26) aufweist, mittels der dieses fließfähige Medium transportiert wird, wobei die Leitungseinrichtung (26) wenigstens abschnittsweise als ein im Inneren des Strahlungselements (22) verlaufender Kanal ausgebildet ist.

8. Verfahren nach Anspruch 7, **dadurch gekennzeichnet, dass** das fließfähige Medium zeitweise zwischen einer Außenwandung des Strahlungselements (22) und einer Innenwandung der Mündung der Behältniskomponente (10) transportiert wird.

## Claims

1. An apparatus (1) for the sterilization of container components (10, 20) with a conveying device (2) which conveys a plurality of sterilization units (8) along a pre-set conveying path (P), wherein each of these sterilization units (8) has a radiation element (22) which is designed in the manner of a rod, which is capable of being introduced into the container component at least in part through an opening (10a) in the container component (10, 20), and wherein the radiation element (22) has at least one radiation source (24) for the emission of ultraviolet (UV) light, **characterized in that** the radiation element has a conducting device (26) for conducting a flowable medium and at least one opening (28, 28a, 28b ...) by means of which the flowable medium can flow between the radiation element (22) and the interior of the container component (10, 20), wherein the conducting device (26) is designed at least in sections in the form of a channel extending in the interior of the radiation element (22).

2. An apparatus (1) according to claim 1, **characterized in that** a cross-section of the radiation element (22) is dimensioned in such a way that when the radiation element is introduced into the container component (10, 20) an outer wall of the radiation element (22) and a wall of the container component (10, 20) have formed between them a gap (42) through which the flowable medium can flow between an interior of the container component (10, 20) and a space situated on the outside with respect to the container component (10, 20).

3. An apparatus (1) according to claim 2, **characterized in that** the gap (42) is an annular gap.

4. An apparatus (1) according to at least one of the preceding claims, **characterized in that** at least one opening (28a, 28b) is arranged in a peripheral face of the radiation element (22).

5. An apparatus (1) according to at least one of the preceding claims, **characterized in that** at least one radiation element (22) has arranged on it a sleeve body (40) which surrounds this radiation element (22) at least in sections.

6. An apparatus (1) according to claim 5, **characterized in that** a gap (44) through which the flowable medium is capable of being conveyed is formed between the sleeve body (40) and the radiation element (22).

7. A method of sterilizing container components (10, 20), wherein the container components (10, 20) are conveyed by means of a conveying device (2) and are sterilized by means of a plurality of sterilization units (8) during this conveying, wherein these sterilization units (8) have in each case radiation elements (22) which are introduced into the container components (10, 20) by way of an opening of the container components (10, 20) and wherein these radiation elements (22) in the interior of the container components (10, 20) emit an ultraviolet radiation for the sterilization of the inner wall of the container components (10, 20), **characterized in that** a flowable medium is supplied to the inner wall of the container components (10, 20), and the radiation element (22) has a conducting device (26) by means of which this flowable medium is conveyed, wherein the conducting device (26) is designed at least in sections in the form of a channel extending in the interior of the radiation element (22).

8. A method according to claim 7, **characterized in that** the flowable medium is conveyed for a time between an outer wall of the radiation element (22) and an inner wall of the opening of the container component (10).

## Revendications

1. Dispositif (1) de stérilisation de composants de récipient (10, 20), avec une installation de transport (2) convoyant une pluralité d'unités de stérilisation (8) le long d'un chemin de transport (P) défini, chacune desdites unités de stérilisation (8) comportant un élément d'irradiation (22) réalisé en forme de barre, lequel est au moins partiellement insérable dans le composant de récipient par l'embouchure (10a) du composant de récipient (10, 20), et où l'élément d'irradiation (22) comprend au moins une source de rayonnement (24) pour l'émission de lumière ultraviolette (UV),
**caractérisée en ce que**
l'élément d'irradiation comprend un dispositif de conduite (26) pour l'amenée d'un agent fluide et au moins une ouverture (28, 28a, 28b..) par laquelle l'agent fluide peut s'écouler entre l'élément d'irradiation (22) et l'intérieur du composant de récipient (10, 20), le dispositif de conduite (26) étant au moins en sections réalisé comme un canal s'étendant à l'intérieur de l'élément d'irradiation (22).

2. Dispositif (1) selon la revendication précédente, **caractérisée en ce qu'**une section transversale de l'élément d'irradiation (22) est dimensionnée de telle manière que, lorsque l'élément d'irradiation est inséré dans le composant de récipient (10, 20), un interstice (42) est formé entre une paroi intérieure de l'élément d'irradiation (22) et une paroi du composant de récipient (10, 20), par lequel l'agent fluide peut s'écouler entre l'intérieur du composant de récipient (10, 20) et un espace extérieur par rapport au composant de récipient (10, 20).

3. Dispositif (1) selon la revendication 2, **caractérisée en ce que** l'interstice (42) est un interstice annulaire.

4. Dispositif (1) selon au moins une des revendications précédentes, **caractérisée en ce qu'**au moins une ouverture (28a, 28b) est ménagée dans une surface périphérique de l'élément d'irradiation (22).

5. Dispositif (1) selon au moins une des revendications précédentes, **caractérisée en ce qu'**est disposé contre au moins un élément d'irradiation (22) un corps en forme de manchon (40) entourant au moins partiellement ledit élément d'irradiation (22).

6. Dispositif (1) selon la revendication 5, **caractérisée en ce qu'**un un interstice (44) est ménagé entre le corps en forme de manchon (40) et l'élément d'irradiation (22), par lequel peut être conduit l'agent fluide.

7. Procédé de stérilisation de composants de récipient (10, 20), lesdits composants de récipient (10, 20) étant convoyés par un dispositif de transport (2) et stérilisés pendant ce transport au moyen d'une pluralité d'unités de stérilisation (8), lesdites unités de stérilisation (8) comportant des éléments d'irradiation (22) respectifs insérés dans les composants de récipient (10, 20) par une embouchure des composants de récipient (10, 20), et lesdits éléments d'irradiation (22) émettant un rayonnement ultraviolet à l'intérieur des composants de récipient (10, 20) pour la stérilisation de la paroi intérieure des composants de récipient (10, 20),
**caractérisée en ce qu'**
un agent fluide est conduit à l'intérieur des composants de récipient (10, 20) et **en ce que** l'élément d'irradiation (22) comprend un dispositif de conduite (26) permettant le transport dudit agent fluide, l'installation de conduite (26) étant au moins en partie réalisé comme un canal s'étendant à l'intérieur de l'élément d'irradiation (22).

8. Procédé selon la revendication 7, **caractérisé en ce que** l'agent fluide est transporté temporairement entre une paroi extérieure de l'élément d'irradiation (22) et une paroi intérieure de l'embouchure du composant de récipient (10).
